# EUROPEAN PATENT APPLICATION

(11) **EP 4 736 823 A1**
(43) Date of publication of application: **06.05.2026**
(21) Application number: 25212337.7
(22) Date of filing: 30.10.2025
(51) Int. Cl.: A61F 13/00, A61F 13/01, A61F 13/36

(54) **WOUND DEBRIDEMENT PAD**

(30) Priority: 31.10.2024 GB 202416083
(71) Applicant: Advanced Medical Solutions Limited, Winsford, Cheshire CW7 3RT (GB)
(72) Inventor: BUGEDO ALBIZURI, Ander, Winsford, CW7 2LF (GB); FERNANDEZ, Robert, Stockport, SK6 2EN (GB)
(74) Representative: Marks & Clerk LLP

(57) **Abstract**

The present invention relates to the debridement of wounds. Specifically, a wound debridement pad is described that comprises one or more woven layers, wherein each woven layer comprises yarns interwoven such that they form periodic clusters of loops, along with cavities between adjacent clusters of loops. The clusters of loops are suitable for removing dead, damaged, or infected tissue from a wound, and the cavities are suitable for collecting the dead, damaged, or infected tissue. A method of using the debridement pad in debriding a wound is also described.

## Description

### FIELD OF INVENTION

The present invention relates to the mechanical debridement of wounds. In particular, a new woven debridement pad is described herein. A method of using the debridement pad is also disclosed.

### BACKGROUND

The mechanical debridement of wounds is an important medical procedure that involves the physical removal of dead, damaged, infected or otherwise non-viable tissue from a wound. It is commonly used in clinical settings but can also be performed at home under medical guidance.

The debridement of a wound has a number of benefits. These include:
Promoting healing of the wound. Debridement can remove non-viable tissue and create a clean environment that facilitates the growth of healthy tissue. The dead tissue may otherwise acts as a barrier, slowing down the natural healing process.
Preventing infection of the patient. Dead or necrotic tissue can harbor bacteria, which increases the risk of infection. By debriding the wound, this source of infection is eliminated, reducing the likelihood of further complications such as cellulitis, abscess formation, or sepsis.
Reducing inflammation. Non-viable tissue can cause prolonged inflammation in the wound area. Removing the non-viable tissue helps reduce chronic inflammation, which can otherwise impede the body's healing response and lead to chronic wounds.
Improving wound assessment. By clearing away dead tissue, clinicians can better evaluate the wound's true depth and extent. This allows for more accurate diagnoses and effective treatment planning.
Decreasing risk of amputation. In serious wounds, especially those with poor circulation (such as diabetic ulcers), infection and poor healing can increase the risk of amputation. Proper debridement helps control infection and enhance healing, lowering the risk of more drastic interventions like amputation.
Enhancing the effectiveness of subsequent treatments. Providing a clean wound bed makes treatments, like dressings and topical medications, more effective. For instance, antibiotics or antiseptic agents can better penetrate a wound free of necrotic tissue, maximizing their efficacy.
Promoting the growth of new tissue. Debridement encourages the formation of granulation tissue, which is essential for wound closure. This is a critical part of the healing process, as new, healthy tissue replaces what was removed.
Preventing malodor and discomfort. Necrotic tissue can emit foul odors and cause discomfort for the patient. Debridement can improve patient quality of life by reducing these symptoms.
Stimulating wound healing. The physical action of mechanical debridement can also stimulate the wound healing process. For example, wet-to-dry dressings, when removed, may lightly abrade the wound, promoting blood flow and encouraging the development of new tissue (granulation).
Removing biofilm. In some cases, biofilms (thin layers of bacteria that adhere to the wound surface) can form on chronic wounds and impair healing. Mechanical debridement is one method of physically disrupting and removing biofilms, improving the wound's response to other treatments, such as antibiotics.

A further advantage of mechanical debridement is that it is relatively inexpensive compared to other types of debridement, such as enzymatic or surgical methods. It often uses basic supplies like gauze and saline, making it a practical option, especially in resource-limited settings. Mechanical debridement also offers a non-surgical alternative for patients who are not suitable candidates for surgical debridement due to health conditions or contraindications. It is a less invasive option that can still be effective in promoting healing.

Mechanical debridement can be combined with other debridement techniques, such as enzymatic or autolytic debridement, to enhance overall effectiveness. For example, mechanical debridement may be used to initially clear away surface debris, while other methods are employed for deeper, more precise tissue removal.

The most common techniques used for mechanical debridement include:
Wet-to-Dry Dressings. A saline-soaked gauze is applied to the wound and allowed to dry. When removed, it pulls off dead tissue along with the dressing.
Irrigation. Pressurized fluids (e.g., saline solution) are used to flush the wound, removing debris and dead tissue.
Scrubbing/Brushing. Gentle scrubbing with a sterile brush or gauze pad to remove necrotic tissue and debris.

The above-mentioned techniques typically suffer from several drawbacks including pain and discomfort to the patient and non-selective removal of tissue (i.e. the debridement removes both necrotic and viable tissue). The techniques can also damage healthy tissue if not done carefully. However, despite these considerations, mechanical debridement remains a valuable and widely used option for wound care, particularly when more advanced or surgical interventions are not necessary or feasible.

Recently there has been the development of a number of tailored debridement pads as a replacement to the sterile brush or gauze pad used in the scrubbing/brushing technique. These debridement pads have protruding fibres (e.g. individual strands or loops) that extend from a carrier or backing layer for abrading the wound and removing the unwanted tissue. These pads are designed to offer gentle abrasion and reduce the discomfort experienced by the patient. Example debridement pads include Debrisoft^{®} by Lohmann & Rauscher, Cutimed^{®} Debriclean by Essity, Prontosan^{®} by Bbraun and Kliniderm^{®} debride by Klinion.

However, it can often be difficult to ensure full removal of the wound debris using such pads. Certain strategies that have therefore been employed to try to improve their effectiveness, including providing areas with shorter stiffer fibres in combination with longer softer fibres (such as those described in the Cutimed^{®} pad and in EP3539517B1). However, this provides an uneven distribution of force, based on the sections with the more abrasive fibres, which provides very little control to the clinician conducting the debridement. It can also make it difficult for a clinician to apply pressure precisely to a desired area of a wound.

Accordingly, there is a need for an alternative debridement pad design aimed at improving the mechanical debridement of wounds.

### SUMMMARY OF THE INVENTION

In general, the present relation relates to a new debridement pad. The debridement pad has a defined woven structure that provides sections of protruding fibres, in the form of clusters of loops that are capable of removing dead, damaged, or infected tissue (wound debris) from a wound, along with cavities capable of collecting the wound debris. The protruding fibres form part of the overall woven structure and therefore, unlike prior art devices, the debridement pad does not require a separate backing or carrier layer. This can cause the article to be more flexible than prior art debridement pads allowing it to be used in a wider range of situations.

Accordingly, in an aspect of the present invention there is provided a wound debridement pad comprising one or more woven layers, wherein each woven layer comprises first yarns extending in a first direction; second yarns extending in a second direction, wherein the first direction is perpendicular to the second direction; and third yarns extending in the first direction, wherein the first yarns and the third yarns are each interwoven with the second yarns such that the third yarns form periodic clusters of loops above and below the first and second yarns, the clusters of loops for removing dead, damaged, or infected tissue from a wound, and wherein cavities exist between adjacent clusters of loops due to an absence of the third yarns, the cavities for collecting the dead, damaged, or infected tissue.

Advantageously, the above-mentioned debridement pad, which comprises periodic clusters of fibre loops (also known as tufts), along with cavities therebetween, provides a tessellating pattern that allows for effective mechanical debridement of wounds by the tufts, coupled with the collection of the wound debris in the cavities. In addition to the collection of the debris in the cavities, the plurality of loops within each tuft, can also allow for entrapment of finer debris in-between the loops. Overall, this can lead to improved removal of debris from a wound compared to known devices, as well as reduced cross contamination.

The interwoven nature of the debridement pad allows for the article to have protruding fibres capable of mechanical debridement without the need for a backing or carrier layer. As shown in the examples, this can provide increased flexibility and structural conformability compared to known devices. This can allow it to be used more effectively on wounds that are on curved body parts.

Advantageously, as shown in the examples below, the debridement pad has also been found to provide an improved transmission of pressure from the user to a wound compared to other known devices. Without wishing to be bound by theory, this is believed to arise due to the lack of the backing layer. The improved transmittance of pressure can allow the user to apply more pressure to the wound, thereby enhancing the wound cleaning efficacy compared to known devices. Alternatively, it can allow the user to achieve the same pressure but with less effort, making the device easier to use for a clinician. The improved transmittance of pressure can also allow the user to better control the pressure in the presence of irregular anatomical features in order to strike an adequate balance between wound cleaning efficacy and control of trauma caused to the patient as a result of the mechanical debridement procedure.

Advantageously, as shown in the examples below, the debridement pad has also been shown to provide higher coefficients of friction than other debridement articles. This can lead to improved removal of debris from a wound and therefore improved debridement performance.

Advantageously, the use of continuous fibres in the form of yarns, rather than discrete fibres attached to and emanating from a carrier layer, as provided in the prior art, may increase the structural integrity of the debridement pad and minimise fibre shredding. This improves the lifetime of the device and reduces the risk of the fibres becoming dislodged from the debridement pad contaminating the wound.

In some embodiments, the fibres of the yarns themselves may also be capable of capturing small debris, e.g. they may be microfibres. This can complement the capture of debris between the loops and within the cavities, thereby further increasing the overall performance. The microfibres can also offer debridement while still feeling soft to the patient.

In another aspect of the invention there is provided the use of the above-mentioned debridement pad in the mechanical debridement of a wound.

In another aspect of the invention there is provided a method of using the above-mentioned debridement pad in debriding a wound, the method comprising: contacting the debridement pad with a wound; applying pressure to the debridement pad; and moving the debridement pad across the wound to remove and collect dead, damaged, or infected tissue from the wound.

The method of using the pad may also comprise applying an aqueous solution, such as a saline solution, to the debridement pad prior to contacting the debridement pad with the wound.

In another aspect of the invention there is provided a packaged article comprising the above-mentioned debridement pad enclosed in packaging, preferably plastic packaging.

In a further aspect of the invention there is provided the use of a cleaning article in the mechanical debridement of a wound, the article comprising one or more woven layers, wherein each woven layer comprises: first yarns extending in a first direction; second yarns extending in a second direction, wherein the first direction is perpendicular to the second direction; and third yarns extending in the first direction, wherein the first yarns and the third yarns are each interwoven with the second yarns such that the third yarns form periodic clusters of loops above and below the first and second yarns, the clusters of loops for removing dead, damaged, or infected tissue from a wound, and
wherein cavities exist between adjacent clusters of loops due to an absence of the third yarns, the cavities for collecting the dead, damaged, or infected tissue.

### DEFINITIONS

As used herein the term "debridement pad" refers to an article that is intended for use in the mechanical debridement of a wound.

As used herein the term "yarn" refers to a continuous strand composed of natural or man-made fibre(s).

As used herein the term "first yarns extending in a first direction" refers to a series of parallel yarns extending along the same direction. These may be a plurality of separate continuous strands, or may be single continuous strand that loops back and forth along the given direction. It will be appreciated that the second and third yarns are defined analogously.

As used herein the term "periodic clusters of loops" refers to series of regularly repeating units, each unit comprising a plurality of loops of yarn positioned adjacent to each other.

As used herein the term "cavity" refers to an area of the pad where there are no loops of the third yarns. The base of the cavity is defined by the interwoven first and second yarns and the depth of the cavity is measured relative to the height of the loops of the third yarns in the adjacent clusters of loops.

As used herein the term "interwoven" refers to two or more yarns that have been weaved together, i.e. where the yarns pass over and under each other to form a woven network.

As used herein the term "perpendicular" refers to a direction that extends at an angle of approximately 90° to another direction.

As used herein the term "monofilament yarns" refers to a yarns composed of a single continuous fibre (i.e. a single fibre that extends along the entire length of the yarn).

As used herein the term "multifilament yarns" refers to yarns comprising a plurality of continuous fibres (i.e. a bundle of fibres that extend along the entire length of the yarn).

As used herein the term "spun yarns" refers to a yarns composed of staple fibres (i.e. discrete lengths of fibres shorter than the length of the yarn) bound together, usually by twisting. It will be appreciated that staple fibres are relatively short fibres, typically ranging from a few inches to a few feet in length, which can be made from natural sources (like cotton or wool) or synthetic materials (like polyester). These fibres are generally gathered, aligned, and then twisted together to form spun yarns.

As used herein the term "microfibres" refers to fibres that are one denier or less. Microfibres typically have a diameter of less than ten micrometres. Microfibres are typically thirty times finer than cotton and ten times finer than silk.

As used herein the term "area number density" refers to the number of a specified object per unit area, for example, the number of clusters of loops (tufts) in a given surface area of the debridement pad.

The unit "denier" is used herein. It will be appreciated that denier is a unit of measurement for the linear mass density of yarn and refers to the mass in grams per 9,000 meters of yarn. To measure denier, 1km of yarn is weighed and divided by 9,000.

The debridement pad above has been described in relation to yarns extending in defined directions, however, it will be appreciated that it could also be described in relation to known weaving terms. For example, the debridement pad may be considered to have a warp/weft structure. In such a definition, the first yarns may be a ground warp, the second yarns may be a weft, and the third yarns may be a pile warp. It will be appreciated that warp yarns (or warp fibres) are continuous threads extended longitudinally on the direction of the machine weaving process, while the weft yarns (or weft fibres) are the continuous threads that are weaved across the width of the machine weaving process interlacing perpendicularly in amongst the warp yarns.

### DETAILED DESCRIPTION

### Composition of the yarns

As described above, the debridement pad according to the present invention comprises one more woven layers comprising first yarns, second yarns and third yarns.

The first, second and/or third yarns may be monofilament yarns, multifilament yarns, and/or spun yarns.

In some preferred debridement pads, the first, second and third yarns are multifilament yarns. In other debridement pads, the first and second yarns are monofilament yarns and third yarns are multifilament yarns. In yet further debridement pads, the first and second yarns are spun yarns and third yarns are multifilament yarns.

The first yarns may comprise synthetic fibres. For example, the first fibres may comprise nylon, polyester, polyamide and/or rayon. Alternatively, the first yarns may comprise naturally-derived fibres. For example, the first yarns may comprise lyocell, silk, bamboo fibres, linen, cotton, wool, hemp or jute. The first yarns may also comprise a combination of the above-mentioned synthetic and naturally-derived fibres.

Preferably, the first yarns comprise polyester, for example, the first yarns may consist of polyester. The first yarns may comprise polyamide, for example, the first yarns may consist of polyamide. The first yarns may comprise polyester and polyamide, for example, the first yarns may comprise 70-90% by weight polyester and 10-30% by weight polyamide.

The second yarns may comprise synthetic fibres. For example, the second yarns may comprise nylon, polyester, polyamide and/or rayon. Alternatively, the yarns may comprise naturally-derived fibres. For example, the second yarns may comprise lyocell, silk, bamboo fibres, linen, cotton, wool, hemp or jute. The second yarns may also comprise a combination of the above-mentioned synthetic and naturally-derived fibres.

Preferably, the second yarns comprise polyester, for example, the second yarns may consist of polyester. The second yarns may comprise polyamide, for example, the second yarns may consist of polyamide. The second yarns may comprise polyester and polyamide, for example, the second yarns may comprise 70-90% by weight polyester and 10-30% by weight polyamide.

The third yarns may comprise synthetic fibres. For example, the third yarns may comprise nylon, polyester, polyamide and/or rayon. Alternatively, the third yarns may comprise naturally-derived fibres. For example, the third yarns may comprise lyocell, silk, bamboo fibres, linen, cotton, wool, hemp or jute. The third yarns may also comprise a combination of the above-mentioned synthetic and naturally-derived fibres.

Preferably, the third yarns comprise polyester. The third yarns may comprise polyamide. Further preferably, the third yarns comprise polyester and polyamide. Further preferably, the third yarns comprise 70-90% by weight polyester and 10-30% by weight polyamide. Even further preferably, the third yarns comprise 75-85% by weight polyester and 15-25% by weight polyamide. For example, the third yarns may comprise 80% by weight polyester and 20% by weight polyamide. The third yarns may consist of polyester and polyamide.

In particularly preferred debridement pads, the first and second yarns comprise, preferably consist of, polyester, and the third yarns comprise, preferably consist of, 70-90% by weight polyester and 10-30% by weight polyamide. In another preferred debridement pad, the first and second yarns comprise, preferably consist of, polyester, and the third yarns comprise, preferably consist of, 75-85% by weight polyester and 15-25% by weight polyamide. In another preferred debridement pad, the first and second yarns consist of polyester and the third yarns consist of 80% by weight polyester and 20% by weight polyamide
The combination of polyester and polyamide offers an advantageous combination of properties to the debridement pad. The polyester is lyophilic attracting dirt and oils, while the polyamide is hydrophilic, absorbing water and other fluids.

Preferably, the third yarns comprises microfibres. The first and second yarns may also comprise microfibres. Alternatively, the first and second yarns may not comprise microfibres. In particularly preferred debridement pads, the third yarns comprise microfibres but the first and second yarns do not comprise microfibres.

The microfibres may be split microfibres, e.g. the microfibres may comprise cavernous openings in the fibre. Split microfibres are typically obtained by undergoing a splitting process where the fibre is corroded under controlled chemical conditions. A split microfibre may contain more than 200 million openings per square inch of fabric.

Advantageously, microfibres, and particularly split microfibres, can display greater absorbency than conventional cotton products due to the greater surface area of the fabric making it possible to trap more wound debris. Microfibre fabrics can have as many as 200,000 fibres per square inch and thus 40 times more cloth surface. The entrapment of wound debris within the fibres themselves complements the trapping of debris within both the cavities and between the individual loops of each tuft, thereby providing a superior textile arrangement to remove dirt and slough. Microfibres can also be more tightly woven than regular fibres with more fibres per unit area leading to greater durability.

Preferably, the first yarns have a linear mass density of from 50 to 650 denier. For example, the first yarns may have a linear mass density of from 100 to 600 denier. Further preferably, the first yarns have a linear mass density of from 100 to 300 denier. Even further preferably, the first yarns have a linear mass density of from 150 to 250 denier. The first yarns may have a linear mass density of from 175 to 225 denier, such as about 200 denier.

Preferably, the second yarns have a linear mass density of from 50 to 650 denier.. For example, the second yarns may have a linear mass density of from 100 to 600 denier. Further preferably, the second yarns have a linear mass density of from 100 to 300 denier. Even further preferably, the second yarns have a linear mass density of from 100 to 200 denier. The second yarns may have a linear mass density of from 125 to 175 denier, such as about 150 denier.

Preferably, the third yarns have a linear mass density of from 50 to 650 denier. For example, the third yarns may have a linear mass density of from 100 to 600 denier. Further preferably, the third yarns have a linear mass density of from 100 to 300 denier. Even further preferably, the third yarns have a linear mass density of from 100 to 200 denier. The third yarns may have a linear mass density of from 135 to 185 denier, such as about 160 denier.

### Clusters of loops in woven layer

In the debridement pad according to the present invention, the third yarns form periodic clusters of loops above and below the first and second yarns.

Preferably, each periodic cluster of loops comprises from 2 to 10 loops. Each periodic cluster of loops may comprise from 3 to 8 loops. Each periodic cluster of loops may comprise from 3 to 6 loops.

Preferably, the periodic clusters of loops all comprise the same number of loops. For example, each periodic cluster of loops may consist of 4 loops.

Preferably, the periodic clusters of loops each have a length measured along the first (e.g. warp) direction of from 0.01 to 1 cm. For example, the periodic clusters of loops may each have a length measured along the first direction of from 0.05 to 0.7 cm. Further preferably, the periodic clusters of loops each have a length measured along the first direction of from 0.1 to 0.5 cm. For example, the periodic clusters of loops may each have a length measured along the first direction of from 0.1 to 0.4 cm. The periodic clusters of loops may each have a length measured along the first direction of from 0.2 to 0.5 cm. Even further preferably, the periodic clusters of loops may each have a length measured along the first direction of from 0.2 to 0.4 cm. For example, the periodic clusters of loops may each have a length measured along the first direction of from 0.2 to 0.35 cm. The periodic clusters of loops may each have a length measured along the first direction of from 0.25 to 0.4 cm. The periodic clusters of loops may each have a length measured along the first direction of from 0.25 to 0.35 cm.

Preferably, the periodic clusters of loops each have a length measured along the second (e.g. weft) direction of from 0.05 to 2 cm. For example, the periodic clusters of loops may each have a length measured along the second direction of from 0.1 to 1.5 cm. The periodic clusters of loops may each have a length measured along the second direction of from 0.1 to 1 cm. The periodic clusters of loops may each have a length measured along the second direction of from 0.2 to 1 cm. The periodic clusters of loops may each have a length measured along the second direction of from 0.3 to 1 cm. The periodic clusters of loops may each have a length measured along the second direction of from 0.1 to 0.8 cm. The periodic clusters of loops may each have a length measured along the second direction of from 0.2 to 0.8 cm. Further preferably, the periodic clusters of loops each have a length measured along the second direction of from 0.3 to 0.8 cm. For example, the periodic clusters of loops may each have a length measured along the second direction of from 0.3 to 0.7 cm. The periodic clusters of loops may each have a length measured along the second direction of from 0.35 to 0.65 cm. Even further preferably, the periodic clusters of loops may each have a length measured along the second direction of from 0.4 to 0.6 cm. For example, the periodic clusters of loops may each have a length measured along the second direction of from 0.45 to 0.55 cm.

Preferably, loops within the periodic clusters of loops extend above the first and second yarns by a distance of from 1 to 5 mm. It will be appreciated that by "extend above the first and second yarns" by a set distance, refers to the distance between the woven network of the first and second yarns and the apex of the loops of the third yarns in the as manufactured state.

Due to the over and under nature of the weave, it will be appreciated that the periodic clusters of loops would typically extend below the first and second yarns by the same distance. Accordingly, loops within the periodic clusters of loops preferably extend below the first and second yarns by a distance of from 1 to 5 mm.

The periodic clusters of loops may extend above and/or extend below the first and second yarns by a distance of from 0.5 to 10 mm. The periodic clusters of loops may extend above and/or extend below the first and second yarns by a distance of from 1 to 4 mm. The periodic clusters of loops may extend above and/or extend below the first and second yarns by a distance of from 1 to 3 mm. The periodic clusters of loops may extend above and/or extend below the first and second yarns by a distance of from 1.5 to 2.5 mm.

The loops may also be measured when pulled taut (i.e. under tension as described in the examples section below). Accordingly, the loops in the periodic clusters of loops may extend above and/or extend below the first and second yarns when pulled taut by a distance of from 2 to 4.5 mm, preferably, from 2.5 to 3.5 mm.

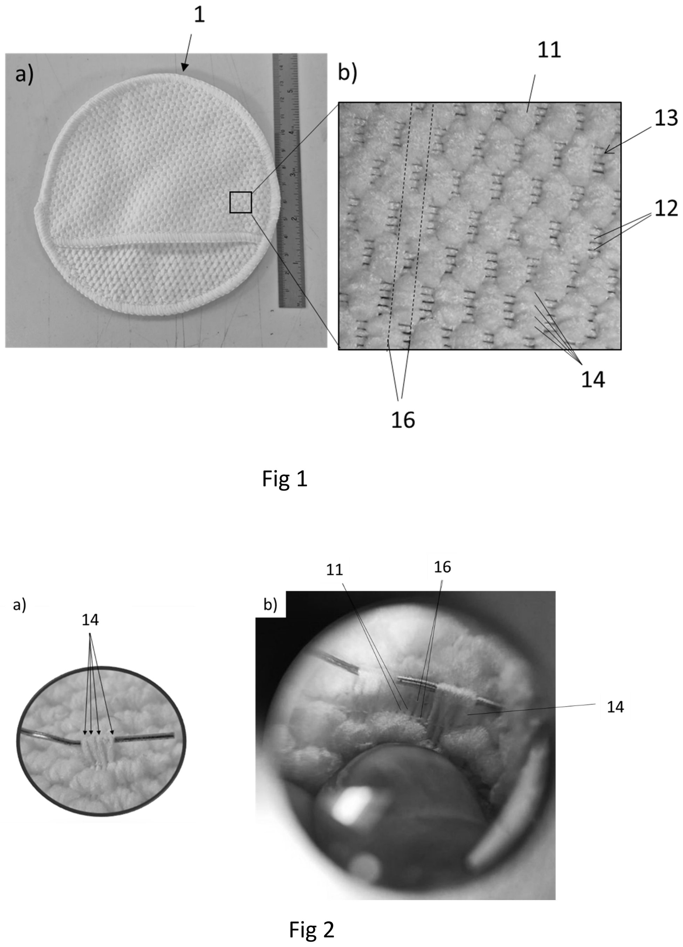

Preferably, the clusters of loops in the debridement pad are generally uniform (i.e. uniform within typical design tolerances) in one or more (e.g. all) size dimensions relative to each other over the majority of the surface area of the debridement pad (e.g. substantially the same length measured along the first direction, and/or substantially the same the length measured along the second direction and/or extend substantially the same the distance above and below the first and second yarns). For example, the clusters of loops in the debridement pad may be generally uniform in their degree of extension above and below the first and second yarns relative to each other over the majority of the surface area of the debridement pad. In this respect, it will be appreciated that the size dimensions may vary within or across an individual cluster (e.g. the shaped clusters shown in figure 1), but that each cluster of loops is generally uniform when compared to other clusters of loops. In embodiments, the clusters of loops in the debridement pad are generally uniform in their size dimensions over essentially all of the surface area of the debridement pad. This is shown, for example, in figure 1 where all clusters of loops are substantially the same over essentially all of the surface area. It will be appreciated in figure 1 that the shape of the clusters at the edges differ merely due to the termination of the pad at edges, otherwise essentially all of the clusters of loops are the same. Advantageously, because of the uniform nature of the clusters of loops (tufts), the abrasive properties of each cluster of loops is substantially the same over the majority of the surface of the debridement pad, providing uniform abrasion properties over the majority of the surface area of the debridement pad. This can allow for more user control and more predictability during debridement of the wound.

Preferably, the individual loops have a total length of from 3 to 10 mm. It will be appreciated the length of an individual loop refers to the length of yarn that forms the loop. It is calculated based on the distance along the yarn in-between the two points the third yarns intersects with the first and second yarns. Further preferably, the individual loops have a total length of from 4 to 9 mm. Even further preferably, the individual loops have a total length of from 5 to 7 mm.

Preferably, the area number density of the clusters of loops is from 100 clusters of loops per 100cm² to 700 clusters of loops per 100cm². Further preferably, the area number density of the clusters of loops is from 250 clusters of loops per 100cm² to 500 clusters of loops per 100cm². The area number density of the clusters of loops may be from 300 clusters of loops per 100cm² to 450 clusters of loops per 100cm².

Preferably, the loops within the periodic clusters of loops are configured to splay apart from each other under pressure to create gaps between the loops for the collection of the dead, damaged, or infected tissue. Advantageously, this splaying allows for the entrapment of dirt in-between the individual loops forming a cluster of loops (tuft).

### Cavities in woven layer

In the debridement pad according to the present invention, cavities exist between adjacent clusters of loops due to an absence of the third yarns.

It will be appreciated that due to the over and under nature of the weave, the size of the cavities can be the same as for the periodic clusters of loops described hereinbefore.

Preferably, each of the cavities have a length measured along the first (e.g. warp) direction of from 0.01 to 1 cm, wherein the length refers to the distance between the loops of adjacent tufts measured along the first direction at the point the third yarns intersects the first and second yarns. Further preferably, each of the cavities have a length measured along the first direction of from 0.1 to 0.5 cm. For example, each of the cavities may have a length measured along the first direction of from 0.2 to 0.4 cm.

Preferably, each of the cavities have a length measured along the second (e.g. weft) direction of from 0.05 to 2 cm, wherein the length refers to the distance between the loops of adjacent tufts measured along the second direction at the point the third yarns intersect the first and second yarns. Further preferably, each of the cavities have a length measured along the second direction of from 0.3 to 0.8 cm. For example, each of the cavities have a length measured along the second direction of from 0.4 to 0.6 cm.

Preferably, each of the cavities have a depth of from 2 to 5 mm. It will be appreciated here that depth refers to the distance between the woven network of the first and second yarns forming the base of the cavity and the apex of the loops of the third yarns forming the adjacent tufts.

### Design of woven layers and overall debridement pad

The debridement pad according to the present invention comprises one or more woven layers.

Preferably, each woven layer of the debridement pad has a basis weight of from 200 to 400 gsm. Further preferably, each woven layer of the debridement pad has a basis weight of from 250 gsm to 350 gsm.

Preferably, the debridement pad has total thickness (i.e. accounting for all woven layers) of from 2 to 20 mm. Further preferably, the debridement pad has total thickness of from 4 to 12 mm. For example, the debridement pad has total thickness of from 6 to 10 mm.

Preferably, the debridement pad comprises two or more of the woven layers. Preferably, the woven layers are attached together. The woven layers may be sewn together. The woven layers may be sewn together using the same fibre material (e.g. having the same composition and denier) as the first, second and/or third yarns. Alternatively, the woven layers may be sewn together using a different fibre material.

Preferably, the wound debridement pad further comprises a pocket for inserting the hand of a user. Particularly preferably, the pocket is formed between two woven layers.

Preferably, the wound debridement pad is configured such that it is reversible. For example, it can be turned inside out and still used for the debridement of wounds. It will be appreciated that the pad being reversible provides more than one configuration suitable for debridement of the wound.

Preferably, the wound debridement pad further comprises an applicator connected to at least one woven layer. The applicator may allow the clinician to apply pressure to the wound through the pad without having to come into direct contact with the woven layers. This may reduce the contamination risk.

As mentioned above, due to the overall woven structure, the debridement pad does not require a separate backing or carrier layer. Accordingly, in preferred debridement pads, the pad does not comprise a backing layer or a carrier layer. The debridement pad may consist essentially of one or more woven layers as herein described. The debridement pad may consist essentially of, preferably consist of, 2 to 5 woven layers attached, preferably sewn, together.

### BRIEF DESCRIPTION OF FIGURES

Figure 1 a) shows a photograph of an exemplary debridement pad according to the present invention. Figure 1b) shows a zoomed in area of the pad of figure 1a).
Figures 2a) and 2b) show photographs of sections of an exemplary debridement pad according to the present invention where clusters of loops have been pulled taut using a metal rod.
Figure 3 shows a lateral cross sectional view of a third yarn forming loops within the present invention.
Figure 4 shows the weaving pattern of an exemplary debridement pad according to the present invention.
Figure 5 shows a microscope image of a single cavity present in an exemplary debridement pad according to the present invention.
Figure 6 shows the reversibility of an exemplary debridement pad according to the present invention. Figure 6a) shows the article in one conformation and figure 6b) shows the same article turned inside out.
Figure 7 shows the experimental set-up used to for frictional testing data shown in figures 8 and 9.
Figure 8 shows the maximum frictional force achieved by an exemplary debridement pad according to the present invention during frictional testing compared to two commercially available debridement pads. Figure 8a) shows the maximum force obtained during testing on dry samples while figure 8b) shows the maximum force obtained during testing on samples which had been hydrated with Solution A.
Figure 9 shows the average frictional force achieved by an exemplary debridement pad according to the present invention during frictional testing compared to two commercially available debridement pads. Figure 9a) shows the average force obtained during testing on dry samples while figure 9b) shows the average force obtained during testing on samples which had been hydrated with Solution A.
Figure 10 shows the experimental set-up used in one method of testing the conformability of debridement pad samples (method 1 in figure 12).
Figure 11 shows the experimental set-up used in another method of testing the conformability of debridement pad samples (method 2 in figure 12).
Figure 12 shows the force required to bend an exemplary debridement pad according to the present invention compared to two commercially available debridement pads, tested using two separate methods.
Figure 13 shows the pressure exhibited by an exemplary debridement pad according to the present invention during pressure testing compared to two commercially available debridement pads. Figure 13a) shows the average pressure exhibited and figure 13b) shows the maximum pressure exhibited.
Figure 14 shows the pressure map recorded during pressure testing of an exemplary debridement pad according to the present invention (Figure 14a) and two commercially available debridement pads (Figures 14b and 14c).
Figure 15 shows the pressure map recorded during testing of an exemplary debridement pad according to the present invention (Figure 15a) and two commercially available debridement pads (Figures 15b and 15c) as part of a finger pointing simulation.
Figure 16 shows the fluid absorbency of an exemplary debridement pad according to the present invention compared to two commercially available debridement pads, tested using Solution A.
Figure 17 shows the results of simulated slough removal using an exemplary debridement pad according to the present invention compared to a commercially available debridement pad.

### EXAMPLES

### Description of exemplary debridement pad

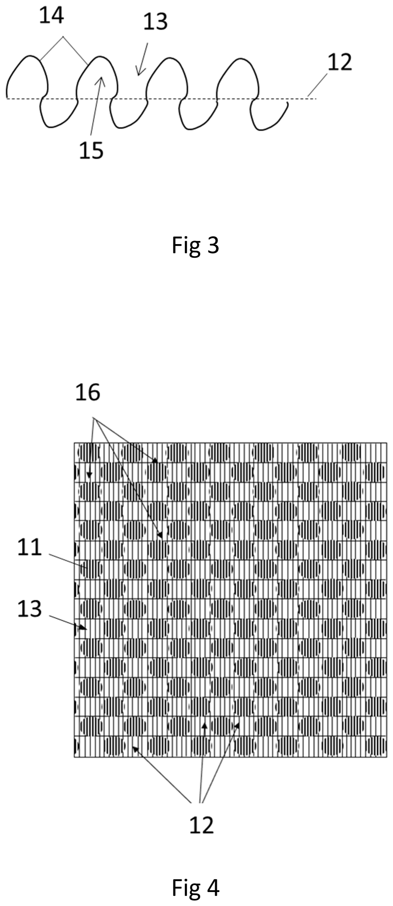

A photograph of an exemplary debridement pad 1 is shown in figure 1. The pad comprises continuous yarns arranged in an interlocking warp/weft configuration such that there is a ground (or structural) warp, a pile warp and a weft. The ground warp consists of 200D (D=denier) polyester fibres, the weft consists of 150D polyester fibres, and the pile warp comprises 160D/72F microfibres (F = number of filaments in a multifilament yarn) made of 80% polyester and 20% polyamide. The weave is designed such that the fibres from the pile warp form periodic clusters **11** of loose loops **14** both above and below the interwoven ground warp **12** and weft **16** (n.b. the weft is not visible in figure 1 but its position has been indicated by dotted line **16).** Figures 2a) and b) show a closer view of a section of the pad where selected clusters of loose loops **11** (herein referred to as tufts) have been pulled taut using a metal rod. As can be seen best in figure 2a), in this exemplary debridement pad, a single cluster (tuft) comprises four closely arranged loose loops. The weft fibres **16** can be seen in figure 2b), which are hidden underneath the tufts in the figure 1. Due to the weaving pattern, where a cluster of closely arranged loose loops **11** is provided on one side of the woven layer, there is an absence of a tuft **11** on the reciprocal side of the layer. As such, cavities **13** are also provided between tufts on the same side of the layer due to the alternating 'over and under' structure of the weave, forming a tessellating pattern. The over/under structure of the pile warp can be seen in the cross sectional representation provided in figure 3.

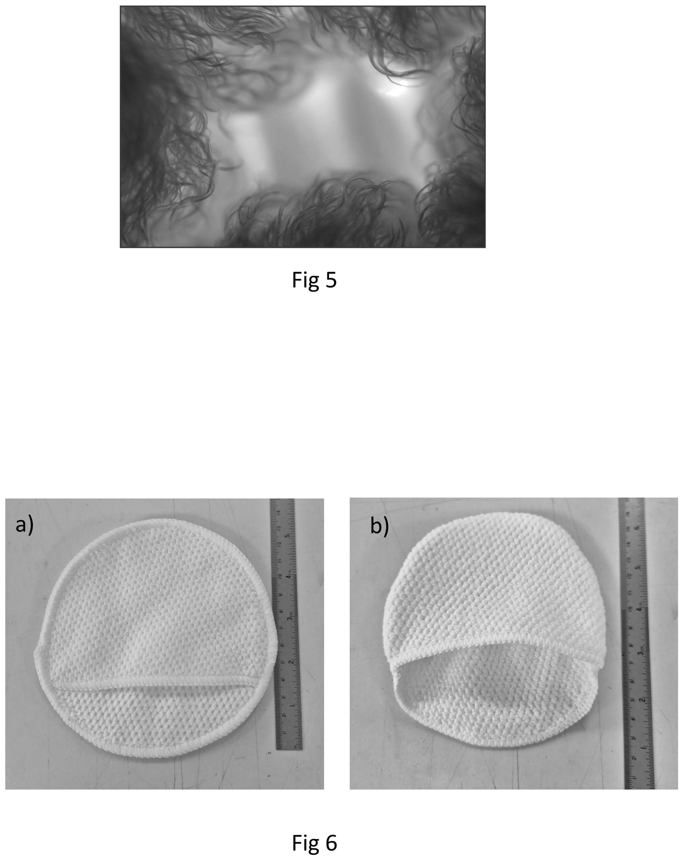

Figure 4 provides a schematic of the weaving pattern showing the ground warp **12,** weft **16** and the tufts **11** that are formed from the pile warp. The tufts **11** are designed to mechanically remove debris from a wound while the cavities **13** are envisaged to be particularly advantageous in collecting wound debris. In particular, the looped multifilament microfibre tufts can maximise surface area friction to remove wound debris and devitalised tissue, whilst still maintaining a gentle feel. The cavities (also known as voids) between the tufts can enhance the debridement through entrapment of the wound debris that gets dislodged through the mechanical friction caused by the microfibre tufts. A microscopic image of one cavity **13** is shown in figure 5. In addition to these cavities, there can also be a hollow **15** provided within each tuft due to the looped nature of the yarns that can allow for further collection of debris and/or for absorption of fluid (figure 3).

Due to the alternating over/under structure of the weave, the article is reversible. This is shown in figures 6a) and 6b) which shows the same article (debridement pad) before and after being turned inside out.

### Dimensions of the clusters of loops / tufts

To quantify the size of the tufts present on the debridement pad, an image of the debridement pad was taken alongside a ruler. ImageJ software was then used with the ruler as a reference to determine the dimensions along the warp (first) direction and weft (second) direction for 25 separate clusters of loops (25 tufts). The median valve was then determined from the 25 measurements.

The number of clusters of loops/tufts present on 10cm of material was also counted along the warp and weft direction (X and Y axis of the image). This was then used to calculate the area number density of the clusters of loops.

The height of the loops (i.e. the distance the loops extend above the first and second yarns in the pad in its as manufactured state) was measured by placing a calibrated ruler perpendicular to the loop and measuring the height using the calibrated ruler.

The height of loops when pulled taut (under tension) was measured by introducing a small metal rod through a loop in order to extend the loop upwards. A calibrated ruler was then placed perpendicular to the loop and the height of the loop was measured against the calibrated ruler. The height under tension provides a rough indication of the length of fibre forming each loop.

The determined dimensions are provided in the table 1 below.

**Table 1 - Dimensions of the repeating clusters of loops present in the debridement pad.**

| **Parameter** | **Results** |
|---|---|
| No. of pile warp fibres (yarns) per cluster of loops/tuft | 4 |
| No. of cluster of loops/tuft per 10cm | 14-15 on weft yarn direction |
| | 26-27 on warp yarn direction |
| Area number density of the clusters of loops/tufts | 385±21 tufts/100cm^{2.} |
| Dimensions of each cluster of loops/tuft (median) | 5.07 mm on weft yarn direction |
| | 2.84 mm on warp yarn direction |
| Height of loops in each cluster of loops / tuft (as manufactured state) | 1.5-2.5 mm |
| Height of loops in each cluster of loops /tuft (pulled taut) | 3-3.5 mm |

The overall mass per area (i.e. basis weight) of each woven layer of the pad, determined by weighing a sample of a woven layer with a known surface area on a gravimetric balance, was 290±5 gsm.

### Method of manufacturing the debridement pad

An exemplary method of manufacturing the debridement pad involves a double warp thread sequence. The first warp acts as the ground structure and the second warp forms the alternating pile. The weaving arrangement therefore results in three different yarns: a structural or ground warp, a pile warp and a weft. Spools of a polyester yarn for the ground warp and polyester/polyamide yarn for the pile warp are threaded into warping beams forming a spread of warp yarns arranged in parallel; these are threaded in pairs to form the ground and pile structure. The warping beams are installed into the loom. A first beam is used to thread the ground warp and a second beam is used to form the pile. Both beams are then independently operated to allow for the perpendicular inter-threading of the weft yarns to form the woven structure; the loom maintains the beam containing the ground warp yarns under tension whilst the beam controlling the pile warp yarns is programmed to lay the yarns in a loose manner according to the specific pattern design.

After the woven material has been prepared, it is cut to a predetermined size and shape, such as a 13cm diameter circle. Two of these cut materials are then stitched together at their extremities. A third piece of cut material is then used to form a pocket for the clinician's hand. The debridement pad is then introduced into a pouch which is sealed to form a sterile barrier. Pouches can then be introduced into cartons. The packaged product is terminally sterilised and subsequently distributed for clinical use.

### Testing compared to commercially available debridement pads

The performance of the woven debridement pad described above, hereinafter referred to as example 1 (E1), was tested against two commercially available debridement pads that comprise arrays of fibres extending from a separate carrier layer. Comparative example 1 (C1), the Debrisoft^{®} pad commercially available from Lohmann and Rauscher, contained a uniform array of fibres over the entire pad, while comparative example 2 (C2), Cutimed^{®} DebriClean commercially available from Essity, contained areas of more abrasive fibres and less abrasive fibres. Neither comparative pad contained any significant cavities.

### Coefficient of friction

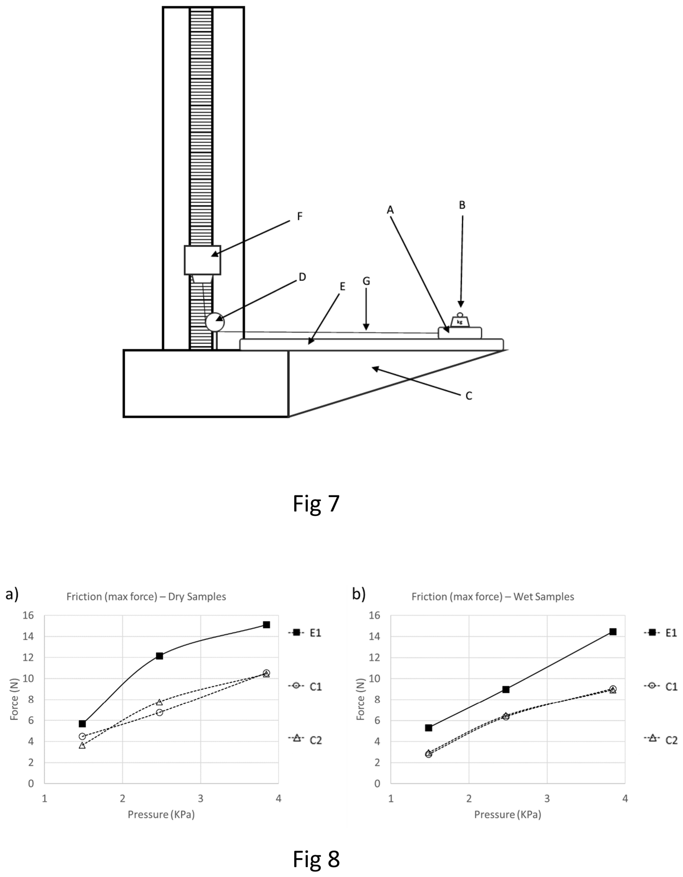

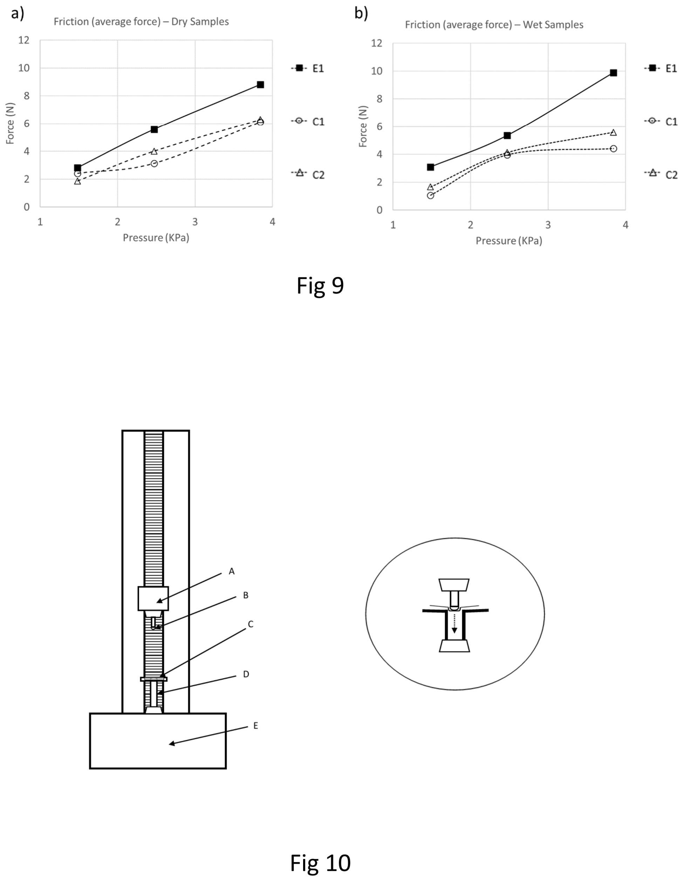

A rectangular sample of each of E1, C1 and C2 was placed on a coefficient of friction pulley pad with a weight on top and dragged over a fake skin surface (Professional Skin Pad mk2 commercially available from limbsandthings). The maximum and average forces are recorded using a Zwick tensometer, employing Testxpert II software and a 50N load cell. A diagram of the experimental set-up is provided in figure 7, which shows pulley pad A, weight B, coefficient of friction attachment C, low friction pulley D, fake skin surface E, load cell F and nylon monofilament G. The pulley pad had a contact surface area of 6.2mm x 6.2mm. Three different weights (600g, 1000g and 1555g), each with a rectangular 5 x 5 cm cross section, were used to evaluate the frictional force generated under different pressures. Each sample was tested both dry and hydrated with solution A. Solution A consists of 142 millimoles of sodium ions and 2.5 millimoles of calcium ions dissolved in distilled water and is deemed an analogue to saline solution. It is common clinical practice to soak a mechanical debridement pad with a saline solution during the mechanical debridement and wound cleansing process. The solution may aid in the dislodging of wound debris, slough and devitalised tissue, however, clinicians may choose not to do so if the wound is already exhibiting copious amount of exudate. Table 2 and figure 8 shows the maximum force recorded for each sample. Figure 8a) shows the maximum force recorded for the three dry samples and figure 8b) shows the maximum force recorded for the three samples hydrated with solution A. Table 3 and figure 9 shows the average force recorded for each sample. In analogy with figure 8, figure 9a) shows the average force recorded for the three dry samples and figure 9b) shows the average force recorded for the three samples hydrated with solution A.

**Table 2 - maximum force recorded during friction testing**

| **Weight (9)** | | **Maximum Force Recorded (N)** | | | | | |
|---|---|---|---|---|---|---|---|
| | **Pressure (Pa)** | **E1** | | **C1** | | **C2** | |
| | | **Dry** | **Wet** | **Dry** | **Wet** | **Dry** | **Wet** |
| 600 | 1482.48 | 5.69 | 5.32 | 4.49 | 2.81 | 3.66 | 2.97 |
| 1000 | 2470.46 | 12.17 | 9.00 | 6.78 | 6.36 | 7.77 | 6.47 |
| 1555 | 3842.10 | 15.12 | 14.47 | 10.55 | 9.06 | 10.44 | 8.94 |

**Table 3 - Average force recorded during friction testing**

| **Weight (9)** | | **Average Force Recorded (N)** | | | | | |
|---|---|---|---|---|---|---|---|
| | **Pressure (Pa)** | **E1** | | **C1** | | **C2** | |
| | | **Dry** | **Wet** | **Dry** | **Wet** | **Dry** | **Wet** |
| 600 | 1482.48 | 2.84 | 3.10 | 2.42 | 1.06 | 1.87 | 1.67 |
| 1000 | 2470.46 | 5.59 | 5.36 | 3.14 | 3.96 | 4.02 | 4.13 |
| 1555 | 3842.10 | 8.82 | 9.89 | 6.12 | 4.42 | 6.30 | 5.59 |

As can be seen in the data above, E1 exhibited the highest peak frictional forces across all three of the tested weights in both the dry and wet states. E1 also exhibited the highest average forces. Without wishing to be bound by theory, it is believed that the increased friction provides an improved ability to detach debris from wounds and therefore an improved debridement performance.

Advantageously, E1 also did not exhibit a meaningful decrease of peak or average friction profile between the dry and wet states, showing its applicability under different conditions.

### Conformability

Two test methods were employed to test the conformability of the example and comparative debridement pads.

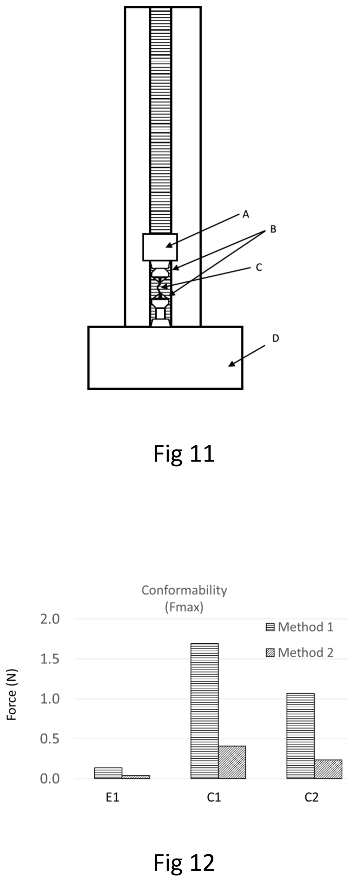

In line with the coefficient of friction testing described above, a Zwick tensometer with Testxpert II software and a 50N load cell was used. In method 1, a ball shaped probe attached to the load cell was pushed against a rectangular shaped sample and towards a circular shaped hole. A diagram of the experimental set-up for method 1 is provided in figure 10, which shows load cell A, ball shaped probe B, rectangular shaped sample C, tubular hollow attachment D and tensometer based E. The forces required to bend and conform samples of each of E1, C1 and C2 into the hole were recorded. In method 2, a rectangular shaped sample (25 x 100 mm) of each of E1, C1 and C2 was secured between two clamps attached to the load cell. The forces required to bend and conform the product were recorded. A diagram of the experimental set-up for method 2 is provided in figure 11, showing load cell A, sample attachment jaws B, rectangular shaped sample C, tensometer based D. For both method 1 and method 2, data was recorded for 10 samples and then averaged. The results are shown in table 4 and figure 12.

**Table 4 - Average force recorded during conformability testing**

| | Method 1 - Force (N) | | | Method 2 - Force (N) | | |
|---|---|---|---|---|---|---|
| Test Number | **E1** | **C1** | **C2** | **E1** | **C1** | **C2** |
| 1 | 0.0398 | 1.5300 | 1.0200 | 0.0176 | 0.4230 | 0.2350 |
| 2 | 0.0437 | 1.6000 | 0.9290 | 0.0368 | 0.4110 | 0.2380 |
| 3 | 0.0346 | 2.2500 | 0.9960 | 0.0366 | 0.4050 | 0.2330 |
| 4 | 0.0408 | 1.7900 | 1.1900 | 0.0375 | 0.4070 | 0.2360 |
| 5 | 0.0363 | 1.4100 | 1.0600 | 0.0381 | 0.4060 | 0.2340 |
| 6 | 0.0499 | 2.0400 | 1.2400 | 0.0388 | 0.4130 | 0.2430 |
| 7 | 0.0394 | 1.8400 | 0.8940 | 0.0395 | 0.4200 | 0.2370 |
| 8 | 0.6270 | 1.3600 | 1.0800 | 0.0393 | 0.4000 | 0.2320 |
| 9 | 0.4210 | 1.6900 | 0.8520 | 0.0389 | 0.4040 | 0.2380 |
| 10 | 0.0348 | 1.4000 | 1.4100 | 0.0394 | 0.4070 | 0.2310 |
| | | | | | | |
| Average | **0.137** | **1.691** | **1.067** | **0.036** | **0.410** | **0.236** |
| Standard Deviation | 0.210 | 0.293 | 0.172 | 0.007 | 0.007 | 0.004 |

As can be seen, less force was required to bend E1 in both test methods compared to both C1 and C2. This demonstrates a higher degree of conformability.

### Pressure Mapping

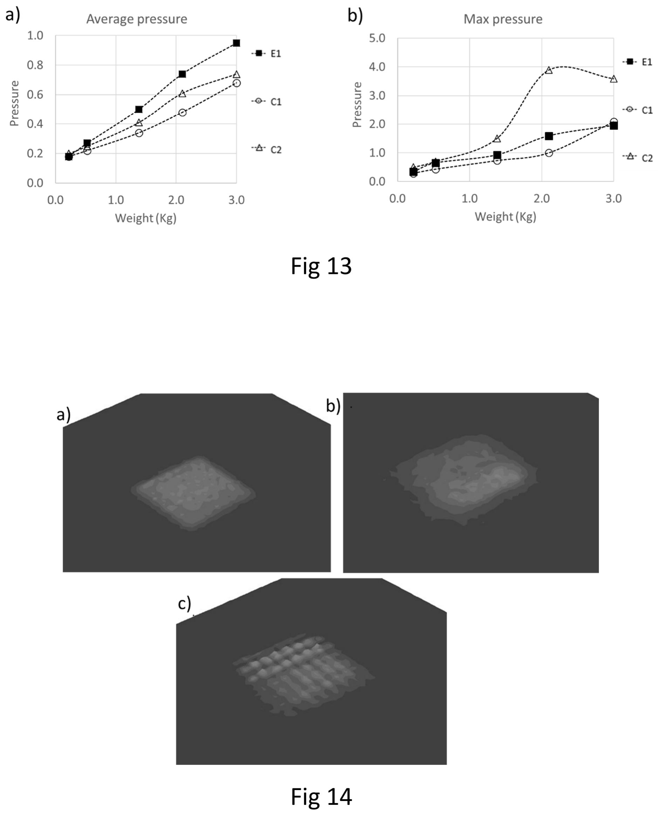

To assess the pressure transferred through each of E1, C1 and C2, a sample was placed on top of an X sensor LX205 100 pressure sensor mat.. A 5 x 5cm plastic rectangle was then placed on top of the sample with a load weight placed on top of the plastic rectangle. The pressure mat recorded the weight pressure exerted by the 5 x 5cm rectangle through each debridement pad sample, along with the cross sectional area where weight pressure could be detected. The area value provides an indication of the extent that the sample disperses the weight applied to it. The results for E1, C1 and C2 are shown in Table 5. The average pressure and the maximum pressure exerted on the pressure mat through the sample is plotted in figures 13a) and 13b) respectively. Figure 14a) shows the pressure map for E1, recorded from the pressure mat using X3 Pro software, b) shows the corresponding pressure map recorded for C1, and c) shows the corresponding pressure map recorded for C2.

**Table 5 - Pressure recorded during pressure mapping testing**

| **E1** | | | |
|---|---|---|---|
| **Weight applied to plastic rectangle (Kg)** | **Area under sample where pressure was detected (cm²)** | **Average pressure exerted though sample (N/cm²)** | **Maximum pressure exerted through sample (N/cm²)** |
| 0.221 | 15.16 | 0.18 | 0.34 |
| 0.525 | 25.16 | 0.27 | 0.65 |
| 1.38 | 33.03 | 0.50 | 0.93 |
| 2.1 | 34.26 | 0.74 | 1.59 |
| 3 | 35.35 | 0.95 | 1.95 |
| | | | |

| **C1** | | | |
|---|---|---|---|
| **Weight applied to plastic rectangle (Kg)** | **Area under sample where pressure was detected (cm²)** | **Average pressure exerted (N/cm²)** | **Maximum pressure exerted (N/cm²)** |
| 0.221 | 6.19 | 0.18 | 0.29 |
| 0.525 | 23.29 | 0.22 | 0.43 |
| 1.38 | 44.19 | 0.34 | 0.73 |
| 2.1 | 48.45 | 0.48 | 1.00 |
| 3 | 48.97 | 0.68 | 2.08 |
| | | | |

| **C2** | | | |
|---|---|---|---|
| **Weight applied to plastic rectangle (Kg)** | **Area under sample where pressure was detected (cm²)** | **Average pressure exerted (N/cm²)** | **Maximum pressure exerted (N/cm²)** |
| 0.221 | 9.61 | 0.20 | 0.5 |
| 0.525 | 22.90 | 0.25 | 0.7 |
| 1.38 | 35.48 | 0.41 | 1.5 |
| 2.1 | 41.03 | 0.61 | 3.89 |
| 3 | 45.87 | 0.74 | 3.59 |

As seen in the data above, particularly looking at the higher weights, E1 has a greater ability to transmit force on average and this is transmitted in a more focalised manner (i.e. the force is distributed over a smaller area). Without wishing to be bound by theory, this is believed to arise due to the construction of the pad that does not require a carrier or backing layer that can act to distribute the weight. Advantageously, this can mean that the clinician has a higher level of control of the pressure being applied to various parts of the wound. This is particularly desirable for wounds on uneven surfaces, such as on limbs, that may require different amounts of force being applied at different locations. It also allows the clinician to focus the force on areas of the wound that require the most debridement, and to alter the pressure easily based on feedback from the patient in relation to their pain threshold.

Regarding the maximum pressure readings, C2 shows higher max pressures than E1 or C1. This is believed to arise due to the more rigid abrasive fibres present in a portion of the product surface. Whilst the peak forces are higher in this case, it provides an uneven distribution of force, based on the sections with the more abrasive fibres, which provides very little control to the user.

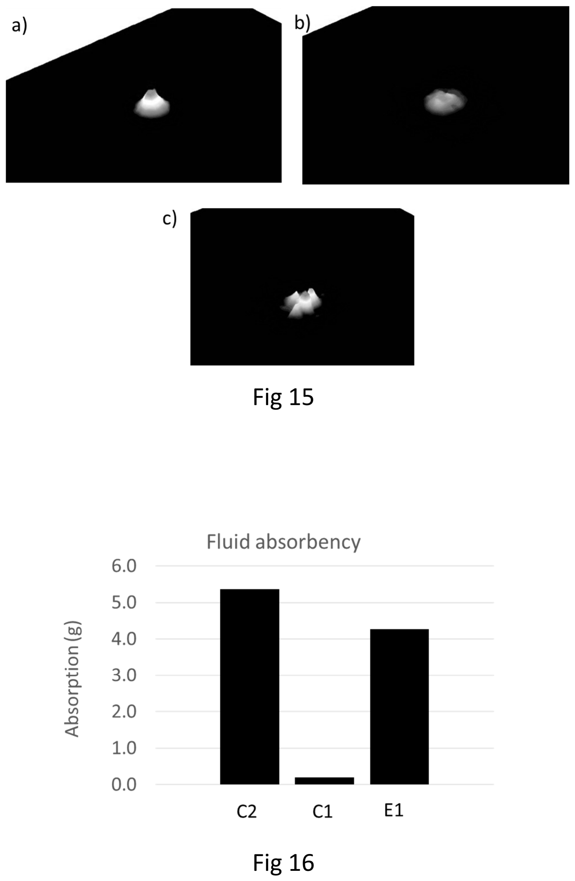

A further pressure measurement was also conducted on each of E1, C1 and C2 to simulate the use of a finger. Each sample was placed on top of a pressure mat and a 2.1 kg spherical ball was put on top to simulate a finger trying to apply focalised pressure through the debridement pad. The distribution of the weight exerted by the spherical ball through the debridement pads was recorded. The sample size used was significantly larger than the area being tested by the ball and therefore not relevant to the measurement. The results are shown in table 6 and figure 15. Figure 15a) shows the pressure map for E1, b) shows the pressure map for C1, and c) shows the pressure map for C2.

**Table 6 - Pressure recorded during finger pointing simulation**

| **Sample** | **Weight (Kg)** | **Area (cm²)** | **Average Pressure (N/cm²)** | **Maximum Pressure (N/cm²)** |
|---|---|---|---|---|
| **E1** | 2.1 | 10.13 | 1.92 | 11.03* |
| **C1** | 2.1 | 28.58 | 0.79 | 4.87 |
| **C2** | 2.1 | 20.45 | 1.14 | 11.03* |

| | | | | |
|---|---|---|---|---|
| *Maximum pressure reading reached | | | | |

Again, E1 exhibited the best transfer of pressure through the pad. This is demonstrated by the focalised transfer of the force with the highest average pressure and lowest surface area pressure distribution. This may allow a clinician to apply pressure more precisely to the desired area of a wound.

### Fluid Absorbency

To measure the fluid absorbency samples of each of E1, C1 and C2 were cut using a 4.75 x 4.75 cm die cutter. The dry weight of the cut samples were then weighed using a balance. Each sample was then immersed in 100ml of Solution A (as defined above) for 30 seconds to saturate the sample. Samples were then removed from the Solution A and allowed to drip excess fluid for 30 seconds. The weight of the hydrated sample weight was then recorded gravimetrically using a balance. The difference in weight between the hydrated sample and non-hydrated sample was then determined, providing a value for the weight of fluid absorbed. The weight differences in grams for the samples are plotted in figure 16. As can be seen, E1 exhibited a relatively high adsorption of fluid, showing a value significantly higher than C1 and close to C2.

### Simulated Slough Removal

To simulate the removal of slough from a wound, a 6.5 x 10 cm sample mounted on a metal sample holder was pulled along a testing plate containing a slough simulant. In line with the experiments above, a Zwick tensometer with Testxpert II software and a 50N load cell was used. The amount of slough simulant moved off the testing plate (i.e. dispersed from the wound bed) and the amount of slough picked up by the sample was determined gravimetrically. The slough simulant was prepared as follows: 1g protanal was mixed with 62.5 glycerol in a 250ml beaker to form a paste. 7.5g guar gum and 5g carboxymethyl cellulose was added to the mixture and homogenised. 195g Reverse osmosis water was then slowly added while stirring continuously until a thick gel was formed. The gel was then transferred to a petri dish and covered for 30 minutes at ambient conditions.

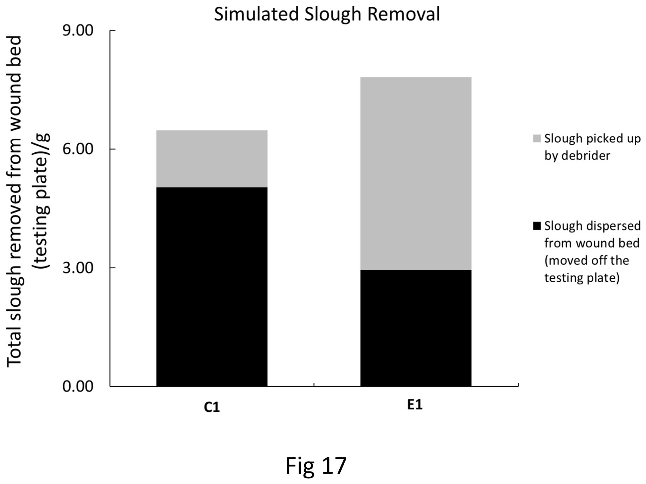

E1 was tested against C1 and the results are shown in figure 17. As can be seen, E1 displayed the highest removal of slough from the wound demonstrating its effective use at debridement of wounds.

## Claims

1. A wound debridement pad comprising:
one or more woven layers, wherein each woven layer comprises:
first yarns extending in a first direction;
second yarns extending in a second direction, wherein the first direction is perpendicular to the second direction; and
third yarns extending in the first direction,
wherein the first yarns and the third yarns are each interwoven with the second yarns such that the third yarns form periodic clusters of loops above and below the first and second yarns, the clusters of loops for removing dead, damaged, or infected tissue from a wound, and
wherein cavities exist between adjacent clusters of loops due to an absence of the third yarns, the cavities for collecting the dead, damaged, or infected tissue.

2. The wound debridement pad of claim 1 wherein the first, second and/or third yarns are monofilament yarns, multifilament yarns, or spun yarns.

3. The wound debridement pad of claims 1 or 2 wherein the first, second and/or third yarns comprise polyester, optionally wherein the third yarns comprise polyester and polyamide, preferably 70-90% by weight polyester and 10-30% by weight polyamide.

4. The wound debridement pad of any preceding claim, wherein the third yarns, and optionally the first and/or second yarns, comprise, preferably consist of, microfibres, such as split microfibres.

5. The wound debridement pad of any preceding claim, wherein the periodic clusters of loops each comprise from 2 to 10 loops, preferably from 3 to 8 loops, further preferably from 3 to 6 loops.

6. The wound debridement pad of any preceding claim, wherein the area number density of the clusters of loops is from 250 clusters of loops per 100cm² to 500 clusters of loops per 100cm².

7. The wound debridement pad of any preceding claim, wherein the loops within the periodic clusters of loops are configured to splay apart from each other under pressure to create gaps for the collection of the dead, damaged, or infected tissue.

8. The wound debridement pad of any preceding claim, wherein the periodic clusters of loops each have a length measured along the first direction of from 0.1 to 0.5 cm; and/or wherein the periodic clusters of loops each have a length measured along the second direction of from 0.3 to 0.8 cm.

9. The wound debridement pad of any preceding claim, wherein loops within the periodic clusters of loops extend above the first and second fibres by a distance of from 1 to 5 mm; and/or wherein loops within the periodic clusters of loops extend below the first and second fibres by a distance of from 1 to 5 mm.

10. The wound debridement pad of any preceding claim, wherein the third yarns have a linear mass density of from 100 to 600 denier, preferably of from 100 to 300 denier.

11. The wound debridement pad of any preceding claim, wherein each woven layer has a basis weight of from 200 to 400 gsm; and/or wherein the pad has total thickness of from 4 to 12 mm.

12. The wound debridement pad of any preceding claim, wherein the pad comprises two or more of said woven layers, and wherein the woven layers are attached together, preferably sewn together.

13. The wound debridement pad of any preceding claim further comprising a pocket for inserting the hand of a user, preferably wherein the pad is the pad of claim 12 and the pocket is formed between two of said woven layers; and/or further comprising an applicator connected to at least one woven layer.

14. The wound debridement pad of any preceding claim where the pad is configured such that it is reversible, thereby providing more than one configuration for debridement of the wound.

15. A packaged article comprising the debridement pad of any one of claims 1 to 14 enclosed in packaging, preferably plastic packaging.
